# EUROPEAN PATENT SPECIFICATION

(11) **EP 2 288 578 B1**
(45) Date of publication and mention of the grant of the patent: **01.02.2012**
(21) Application number: 09762109.8
(22) Date of filing: 04.06.2009
(51) Int. Cl.: C02F 1/32

(54) **PHOTOCHEMICAL REACTOR AND PHOTOCHEMICAL PROCESSING SYSTEM**
PHOTOCHEMISCHER REAKTOR UND PHOTOCHEMISCHES BEARBEITUNGSSYSTEM
RÉACTEUR PHOTOCHIMIQUE ET SYSTÈME DE TRAITEMENT PHOTOCHIMIQUE

(30) Priority: 12.06.2008 EP 08158093
(43) Date of publication of application: 02.03.2011
(73) Proprietor: Koninklijke Philips Electronics N.V., 5621 BA Eindhoven (NL); Philips Intellectual Property & Standards GmbH, 20099 Hamburg (DE)
(72) Inventor: CHITTKA, Uwe, NL-5656 AE Eindhoven (NL); GREUEL, Georg, NL-5656 AE Eindhoven (NL); GRUHLKE, Stefan, NL-5656 AE Eindhoven (NL)
(74) Representative: Bekkers, Joost J.J
(86) International application number: PCT/IB2009/052365
(87) International publication number: WO 2009/150582

(56) References cited:
- EP-A- 0 265 983
- WO-A-00/14830
- WO-A-2006/006129
- RU-C1- 2 223 792
- US-A- 5 965 093

## Description

### FIELD OF THE INVENTION:

The invention relates to a photochemical reactor for a fluid.

The invention further relates to a photochemical processing system.

### BACKGROUND OF THE INVENTION:

Photochemical reactors are known per se and are used for photochemical processing of, for example, a fluid. Both chemical and physical disinfection processes have been known and used over a long period of time for reducing pathogenic organisms such as bacteria, viruses, fungi and protozoa. The chemical processes are largely based on the use of chlorine compounds and ozone. Physical processes, such as filtration, ultrasound, heating or irradiating with ultraviolet-light constitute a smaller burden for the ambient environment. In addition, exposure of water to ultraviolet-radiation (further also indicated as UV-radiation) is a continuous and relatively maintenance-free process. So, the use of photochemical reactors for disinfecting water is increasing, especially since in the developing countries the infrastructure of the municipal water supplies does not keep up with the increasing demand.

During recent years especially the UV efficiency of excimer lamps has increased considerably. Excimer radiation is not re-absorbed by the filling gas of a discharge lamp, and thus the efficiencies which can be achieved with such excimer discharge lamps are comparatively high, even when use is made of noble gasses in the discharge lamp. Xenon turned out to be the most efficient noble gas filling. However, because of the short wavelength of 172 nanometers, this radiation is strongly absorbed by water and thus less attractive for aqueous systems.

Recent developments in luminescent materials enable the use of an excimer lamp together with a luminescent material which produces ultraviolet light at a relatively high efficiency. This is, for example, disclosed in US-patent US 6,398,970B1. This patent describes a device for disinfecting water and discloses a gas discharge lamp comprising a Xenon filling, wherein at least part of the inner walls of the discharge vessel are covered by a phosphor emitting in the UV-C range. In such a device, the UV-radiation has a spectral composition which lies exclusively in the range relevant for disinfecting, i.e. between 230 nanometer and 300 nanometer.

A drawback of the known photochemical reactors is that the efficiency reduces over time and that it is relatively expensive to improve the degraded efficiency.

### SUMMARY OF THE INVENTION:

It is an object of the invention to provide a photochemical reactor in which the efficiency can be improved at relatively low cost.

According to a first aspect of the invention, the object is achieved by means of a photochemical reactor for a fluid, the photochemical reactor comprising:
a vessel comprising the fluid,
a light source for generating UV-radiation and emitting it towards the fluid, and
a luminescent material arranged at least partially between the light source and the fluid for converting at least part of the UV-radiation emitted by the light source into further UV-radiation having an increased wavelength compared to the UV-radiation,
the luminescent material being removably connected to the photochemical reactor and being separate from the light source.

The inventors have found that the use of the excimer gas discharge lamp improves the efficiency of the generation of the ultraviolet radiation - especially in the far UV range (also indicated as VUV). However, a drawback of this improved efficiency is the relatively high flux of UV-radiation which impinges on the luminescent material. This UV-radiation - especially VUV radiation - typically has a relatively short wavelength and thus a relatively high energy per photon. As a result, the use of a light emitter emitting relatively short wavelength UV-radiation causes the luminescent material to degrade faster than in conventional fluorescent lamps, which reduces the efficiency of the photochemical reactor over time. By virtue of the fact that the luminescent material is removably connected to the photochemical reactor and is separate from the light source, the luminescent material may be exchanged with new luminescent material if the current luminescent material has degraded due to the impinging UV-radiation. When, for example, the efficiency of the photochemical reactor has decreased to below, for example, a predetermined level, the luminescent material may be replaced.

In the known device, the luminescent material is arranged on the inner wall of the discharge vessel of the discharge lamp. In such an arrangement, the whole discharge lamp has to be replaced when the efficiency of the photochemical reactor is reduced due to degradation of the luminescent material. This replacement of the discharge lamp may be required even though the efficiency of the light source generating the UV-radiation to be absorbed by the luminescent material has, for example, hardly degraded. In such an event, the improvement of the efficiency of the photochemical reactor is relatively expensive and typically more expensive than necessary. In the photochemical reactor according to the invention, only the luminescent material is replaced, which typically reduces the cost of operating such a photochemical reactor.

A further benefit of the photochemical reactor according to the invention is that it enables a user of the photochemical reactor to choose a specific luminescent material or specific mixture of luminescent materials suitable for the photochemical process which is to be performed in the photochemical reactor during operation. The use of luminescent materials to generate the spectrum of radiation required is already known, for example, from US-patent US 6,398,970B1 as previously described. However, in this known configuration of the photochemical reactor, a single UV-source is present emitting UV-radiation having a spectrum which is determined by the UV-source in combination with the luminescent material applied on a wall of the discharge vessel of the UV-source. As such, the known photochemical reactor is arranged for performing a specific photochemical process. In the photochemical reactor according to the invention the luminescent material is removably connected to the photochemical reactor. In such an arrangement, the luminescent material may be chosen to match the requirements of the current photochemical process which must be performed on the fluid currently present in or flowing through the photochemical reactor. When a different photochemical process is required which, for example, requires UV-radiation having a different spectrum, only the luminescent material or the mixture of luminescent materials needs to be exchanged such that the required spectrum is generated by the combination of the light source and the luminescent material to enable the different photochemical processes to be performed by the photochemical reactor. This improves the flexibility of the use of the photochemical reactor while limiting the cost to implement this flexibility.

The light source may be any light emitter able to emit UV-radiation, for example, a low pressure discharge lamp, a high pressure discharge lamp, a dielectric barrier discharge lamp or, for example, a solid-state light emitting element such as a light emitting diode, a laser diode or an organic light emitting diode.

In an embodiment of the photochemical reactor, the light source is removably connected to the photochemical reactor and is separate from the luminescent material. A benefit of this embodiment is that also the light source may relatively easily be replaced. The light source may, for example, also degrade, thereby reducing the efficiency of the photochemical reactor. By enabling the replacement of the light source that is arranged separate from the luminescent material, only the element required to be replaced to improve the efficiency of the photochemical reactor is actually replaced, thus reducing the replacement cost and/or maintenance cost for the photochemical reactor.

A further benefit of this embodiment is that, due to the fact that both the light source and the luminescent material may individually be replaced, a user may choose the light source to the exact requirements of the photochemical reaction and/or to the exact requirements of the luminescent material. Changing the luminescent material may require the light source to emit a different UV-radiation to provide an efficient conversion of the UV-radiation to the further UV-radiation. In the photochemical reactor according to the invention, both the light source and the luminescent material may be exchanged such that the light source may be selected to, for example, emit light which is efficiently absorbed by the luminescent material to optimize the efficiency of the photochemical reactor. Furthermore, the luminescent material may, for example, only convert a part of the light emitted by the light source into further UV-radiation. The remainder of the UV-radiation emitted by the light source may, for example, contribute to the photochemical reaction in the photochemical reactor. By having both the luminescent material and the light source individually removably connected to the photochemical reactor, a suitable UV-radiation and further UV-radiation may be chosen as is required for the photochemical reaction which is to take place in the photochemical reactor in operation.

In an embodiment of the photochemical reactor, the photochemical reactor comprises a luminescent screen comprising the luminescent material. The luminescent screen is removably connected to the photochemical reactor to enable relatively easy replacement of the luminescent material. The luminescent screen may, for example, be constituted of a material being at least partially transparent to the further UV-radiation, for example, quartz. The luminescent material may be arranged as a layer on the luminescent screen. Alternatively, particles of luminescent materials may be embedded in the luminescent screen. Further alternatively, the luminescent screen may be constituted of luminescent material. The luminescent screen may have any shape suitable to efficiently expose the luminescent material to the UV-radiation emitted by the light source.

In an embodiment of the photochemical reactor, a further fluid may flow between the light source and the luminescent material. This further fluid may, for example, be a cooling gas or cooling liquid which may be used to ensure that the temperature of the luminescent material and/or of the light source does not exceed a predetermined level. Luminescent materials may degrade relatively fast when subjected to relatively high temperatures. By applying the further fluid between the light source and the luminescent material, the further fluid may serve as a cooling fluid limiting the increase in temperature and thus reducing the degradation speed of the luminescent material.

Alternatively, the further fluid may be a fluid which in itself requires a photochemical reaction. In this photochemical reaction in the further fluid, mainly the UV-radiation emitted by the light source may be required to generate the photochemical reaction. In such an arrangement, the UV-radiation emitted by the light source may initially be used to generate the photochemical reaction in the further fluid. The remainder of the UV-radiation (which is not used by the further fluid and which is transmitted by the further fluid) impinges on the luminescent material where at least a part of the impinging UV-radiation is converted to further UV-radiation which, for example, is used in the photochemical reaction in the fluid. The photochemical reaction is arranged for performing two different photochemical reactions in a single reactor. This may be used, for example, in a two-step cleaning process of a fluid, or to clean two different fluids using different photochemical processes.

In an embodiment of the photochemical reactor, the further fluid is identical to the fluid. In such a configuration, the photochemical reactor may have, for example, two reaction chambers in which different photochemical reactions are preformed: one process which is relatively efficient when illumination takes place by the UV-radiation emitted directly by the light source, and a second process which is relatively efficient when illumination takes place by the further UV-radiation (which typically has a longer wavelength) emitted by the luminescent material. Also, this configuration may be used to perform the two-step cleaning process, i.e. the further UV-radiation emitted by the luminescent material is used to have a pre-cleaning step of the fluid, and the UV-radiation emitted by the light source is used to generate the second cleaning step, i.e. thoroughly cleaning the fluid to remove all remaining contamination or bacteria.

In an embodiment of the photochemical reactor, the fluid flows through the photochemical reactor. Such a configuration enables the photochemical reactor to be coupled, for example, in a fluid supply line and continuously clean the flowing fluid by the photochemical process. For example, water supply lines may be coupled to the photochemical reactor according to the invention to clean the water while it is being supplied to the user. This may be done on a large scale, for example, by the supplier of the water. The photochemical reactor may, for example, clean the fluid (for example, water) while it flows via the pipes to a location where the cleaned fluid is needed. Alternatively, this may be done by a consumer who installs the photochemical reactor somewhere in his home to improve the quality of the drinking water in the home.

In an embodiment of the photochemical reactor, the light source is a dielectric barrier discharge lamp (further also referred to as DBD lamp). A benefit of using the DBD lamp, for example, a Xenon DBD lamp, is that it enables a high-efficiency, high-flux UV-light source. Such a discharge lamp may generate UV-radiation at wavelengths down to 172 nm at relatively high efficiencies. Using such a light source has the benefit that the photochemical reactor has a relatively high efficiency for generating the UV-radiation, which may result in, for example, a higher flow rate of the fluid through the photochemical reactor. However, a drawback of this high flux of UV-radiation is that the degradation of the luminescent material is accelerated. This acceleration may require the replacement and/or replenishment of the luminescent material to regain a relatively high efficiency of the photochemical reactor. In the photochemical reactor according to the invention, the luminescent material is removably connected to the photochemical reactor and is separate from the light source. The use of the DBD lamp would allow a highly efficient photochemical reactor, while replacement and/or maintenance to ensure that the efficiency remains within certain limits requires the replacement of only the luminescent material, which may have degraded over time. Thus, the operational cost of such a very efficient photochemical reactor may be reduced.

In an embodiment of the photochemical reactor, the light source is arranged in a protective sheath having a wall at least partially transparent to UV-radiation, the luminescent material being arranged on the wall of the protective sheath. Often a protective sheath is present between the light source and the fluid which, in operation, is exposed to the UV-radiation from the light source. This protective sheath prevents the typically high-voltage discharge lamps from contacting the fluid, thus improving safety. The protective sheath is typically transparent to the UV-radiation such that the fluid may relatively easily be illuminated by a light source inside the protective sheath. By applying the luminescent material on, for example, the inner wall of the protective sheath, the luminescent material may be removed independently of the light source, while a high safety level is maintained. In such a configuration, the protective sheath constitutes the luminescent screen as indicated earlier.

A further benefit of using the protective sheath is that the electrodes, which in a DBD lamp are partially arranged outside the DBD lamp, not necessarily need to be corrosion-safe. When using the protective sheath between the fluid and the light source, the light source is not in contact with the fluid. In the DBD lamp one of the electrodes may be arranged at the outer wall of the discharge lamp, inside the protective sheath. Due to this arrangement, the electrodes are not in contact with the fluid, for example, water, and thus do not need to be corrosion-safe.

The use of the protective sheath further thermally insulates the light source from the vessel comprising the fluid. Often, the fluid is water, which is cleaned using a photochemical reaction. This water typically has a relatively low temperature. When the light source is in contact with the water flowing through the vessel, the temperature inside the light source is partially determined by the flowing water - typically cooling the light source. As a result, the temperature in the light source may be too low, which may reduce the efficiency of the generation of UV radiation in the light source. Furthermore, the reduced temperature of the light source due to the flowing water may cause the light source to switch on more difficultly and/or only after considerable time. Such a delay in switching on of the light source may cause water, which is not cleaned, to pass the photochemical reactor - contaminating the remainder of the water supply. This would be reduced due to the protective sheath which enables the temperature of the light source to be different from the temperature of the fluid flowing through the photochemical reactor.

In an embodiment of the photochemical reactor, the UV-radiation comprises a wavelength below 200 nanometers, and the further UV-radiation comprises a wavelength below 300 nanometers.

The invention also relates to a photochemical processing system for photochemically processing contamination in a fluid, the photochemical processing system comprising the photochemical reactor as claimed in any of the claims and comprising a processor for controlling an intensity of the light emitted by the light source and/or for controlling a speed at which the fluid flows through the photochemical reactor and/or for analyzing the contamination level of the fluid before and/or after processing in the photochemical reactor.

### BRIEF DESCRIPTION OF THE DRAWINGS:

These and other aspects of the invention are apparent from and will be elucidated with reference to the embodiments described hereinafter.

In the drawings:
Figs. 1A and 1B show cross-sectional views of a photochemical reactor according to the invention,
Fig. 2 shows a cross-sectional view of a further embodiment of the photochemical reactor according to the invention,
Fig. 3 shows a cross-sectional view of an even further embodiment of the photochemical reactor according to the invention, and
Fig. 4 shows a photochemical processing system according to the invention.

The Figures are purely diagrammatic and not drawn to scale. Particularly for clarity, some dimensions are exaggerated strongly. Similar components in the Figures are denoted by the same reference numerals as much as possible.

### DETAILED DESCRIPTION OF EMBODIMENTS:

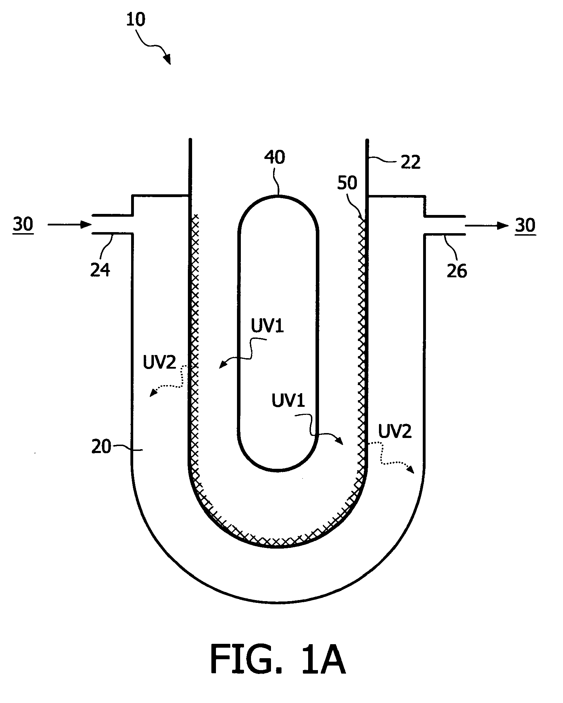

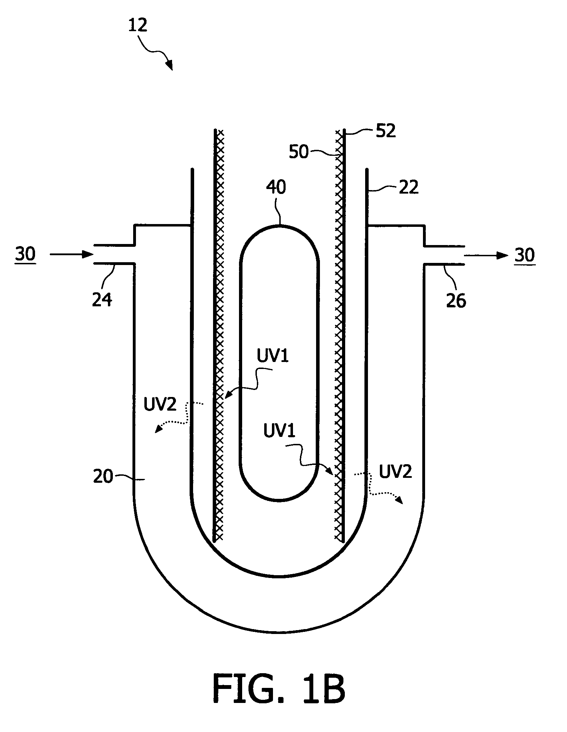

Figs. 1A and 1B show cross-sectional views of a photochemical reactor 10, 12 according to the invention. The photochemical reactor 10, 12 is arranged to photochemically process a fluid 30, and comprises a vessel 20 having a fluid inlet 24 and a fluid outlet 26. During the photochemical processing of the fluid 30, the fluid 30 may flow through the vessel 20, for example, from the fluid inlet 24 to the fluid outlet 26. Alternatively, the fluid 30 may remain stationary in the vessel 20 while being photochemically processed. The photochemical reactor 10, 12 further comprises a light source 40 for generating and emitting ultraviolet radiation UV1 (further also indicated as UV-radiation). The light source 40 may be any light emitter 40 able to emit UV-radiation UV1, for example, a low pressure discharge lamp, a high pressure discharge lamp, a dielectric barrier discharge lamp 40 or, for example, a solid-state light emitting element such as a light emitting diode, a laser diode or an organic light emitting diode. The photochemical reactor 10, 12 further comprises luminescent material 50 arranged at least partially between the light source 40 and the fluid 30. The luminescent material 50 converts at least part of the UV-radiation UV1 emitted by the light source 40 into further UV-radiation UV2 having an increased wavelength compared to the UV-radiation UV1.

The luminescent material 50 may be arranged on a luminescent screen 52. The luminescent material 50 may be applied as a layer on a wall of the luminescent screen 52, for example, between the luminescent screen 52 and the light source 40. In such an arrangement, the luminescent screen 52 is at least partially translucent to at least a part of the further UV-radiation UV2 emitted by the luminescent material. Alternatively, the luminescent material may be applied on a wall of the luminescent screen 52 facing away from the light source 40, or the luminescent material 50 may be embedded in the luminescent screen 52. In such an arrangement, the luminescent screen 52 is at least partially translucent to at least a part of the UV-radiation UV1 emitted by the light source 40, such that the UV-radiation UV1 may impinge on the luminescent material 50 to be converted into further UV-radiation UV2. Still further alternatively, the luminescent screen 52 may be constituted of luminescent material 50.

The luminescent material 50 is removably connected to the photochemical reactor 10, 12 such as to be separate from the light source 40. A benefit of this arrangement is that the luminescent material 50 may be replaced while the remainder of the photochemical reactor 10, 12 can still be used. This may be achieved by replacing the luminescent screen 52 which comprises the luminescent material. Especially when using a DBD lamp 40 as the light source 40, the UV-radiation UV1 may have a wavelength down to 172 nanometers which represents a relatively high energy per photon. Furthermore, the DBD lamps 40 may generate UV-radiation UV1 at a relatively high efficiency and a relatively high flux. This relatively high flux together with the relatively high energy per photon causes the luminescent material 50 to degrade relatively fast. By arranging the luminescent material such that it is removably connected to the photochemical reactor 10, 12, the luminescent material 50 may be replaced without the need to replace the remainder of the photochemical reactor 10, 12. Thus, maintaining the efficiency of the photochemical reactor 10, 12 at a predetermined level may be achieved relatively easily by replacing, for example, the luminescent screen 52.

In the embodiment shown in Fig. 1A, the photochemical reactor 10 comprises a protective sheath 22 which shields the light source 40 from being in contact with the fluid flowing through the photochemical reactor 10. The protective sheath 22 is at least partially translucent to the UV-radiation UV1 emitted by the light source 40 and/or is at least partially translucent to the further UV-radiation UV2 emitted by the luminescent material 50. The protective sheath 22 comprises an inner wall being a wall of the protective sheath 22 facing the light source 40. In the embodiment shown in Fig. 1A, the luminescent material 50 is arranged on the inner wall of the protective sheath 22. A benefit of this embodiment is that the luminescent material 50 is not in direct contact with the fluid 30 and thus cannot be contaminated by the fluid 30. Alternatively, the luminescent material 50 may be embedded in the protective sheath 22 (not shown) or may be applied on an outer wall of the protective sheath 22 being a wall of the protective sheath 22 facing away from the light source 40 (not shown).

In the embodiment shown in Fig. 1A, the light source 40 is arranged separate from the protective sheath 22 and thus separate from the luminescent material 50. Due to this arrangement, the luminescent material 50 and the light source 40 may separately be exchanged. This is very beneficial when the luminescent material 50 and the light source 40 degrade at a different speed with respect to each other. Only the degraded luminescent material 50 (for example, together with the protective sheath 22) has to be replaced, or the degraded light source 40 has to be replaced. Alternatively, this arrangement may be used to select a specific light source 40 to match a specific luminescent material 50. For example, a luminescent material may be chosen because the luminescent material 50 emits the further UV-radiation UV2 having a specific wavelength required for a specific photochemical process. However, the light source 40 currently in use emits the UV-radiation UV1 which cannot efficiently be converted by the luminescent material 50 into the further UV-radiation UV2. In such a situation, the light source 40 may be replaced by a light source 40 emitting UV-radiation UV1 which better matches the requirements of the luminescent material 50. Due to the separate replaceability of both the luminescent material 50 and the light source 40, the flexibility of photochemical reactor 10, in use, is improved.

In the embodiment of the photochemical reactor 12 as shown in Fig. 1B, the luminescent material 50 is arranged on the luminescent screen 52 which is arranged at least partially between the light source 40 and the protective sheath 22. The luminescent screen 52 is removably connected to the photochemical reactor 12. A benefit of this arrangement is that the luminescent screen 52 may be exchanged without exchanging the light source 40 and without replacing the protective sheath 22. Generally, the protective sheath 22 defines part of the flow of the fluid 30 through the photochemical reactor 12. When the luminescent material 50 is arranged on the protective sheath 22, the whole protective sheath 22 must be replaced in order to replace the luminescent material 50. Due to this replacement the fluid must be removed from the photochemical reactor 10. Especially when the photochemical reactor 10, 12 is arranged for decontaminating water, the water will flow through the photochemical reactor 10, 12 at a relatively high pressure. When the protective sheath 22 must be replaced, an additional tap (not shown) is required to (temporarily) block the flow of water until the protective sheath 22 comprising the luminescent material 50 is replaced. In the arrangement as shown in Fig. 1B, the photochemical reactor 12 comprises a luminescent screen 52 comprising the luminescent material 50. The luminescent screen 52 is arranged at least partially between the inner wall of the protective sheath 22 and the light source 40. In this arrangement the luminescent screen 52 may be exchanged on demand without having to remove or replace the protective sheath 22. When the photochemical reactor 12 is used for decontaminating water, the water may remain in the photochemical reactor 12 at its original pressure while the luminescent screen 52 is being replaced relatively easily.

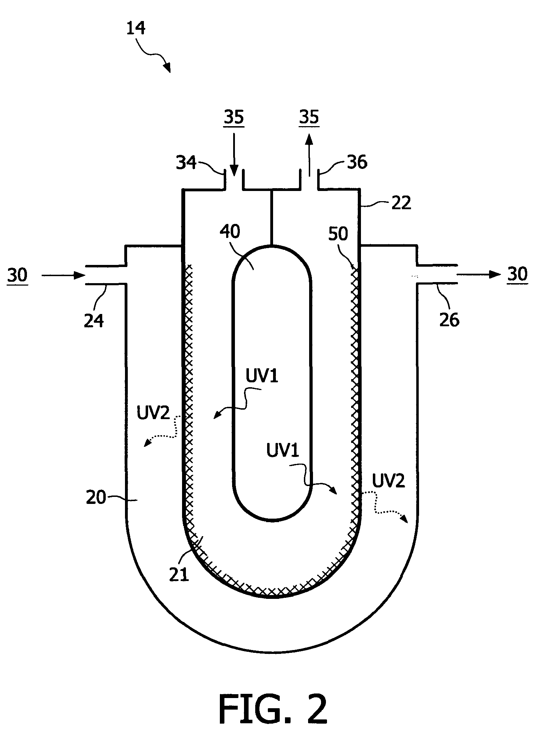

Fig. 2 shows a cross-sectional view of a further embodiment of the photochemical reactor 14 according to the invention. The photochemical reactor 14 shown in Fig. 2 comprises in addition to the fluid inlet 24 and the fluid outlet 26, a further fluid inlet 34 and a further fluid outlet 36. The further fluid inlet 34 provides access to an additional vessel 21 located substantially between the light source 40 and the protective sheath 22. The addition vessel 21 may comprise, in use, a further fluid 35, either flowing through the additional vessel 21 or located in the additional vessel 21. This further fluid 35 may, for example, be a cooling fluid 35 which flows between the light source 40 and the luminescent material 50 to cool the light source 40 and/or the luminescent material 50.

Alternatively, the further fluid 35 may also require photochemical processing, for example, using the UV-radiation UV1 rather than the further UV-radiation UV2. The arrangement as shown in Fig. 2 allows two photochemical processes to take place substantially simultaneously, one process using the UV-radiation UV1 emitted directly by the light source 40 and a further photochemical process using the further UV-radiation UV2 emitted by the luminescent material 50 and thus requiring UV-radiation having a longer wavelength for an efficient photochemical process. Generally, the further fluid 35 should be at least partially translucent to the UV-radiation UV1 emitted by the light source 40 such that part of the UV-radiation may impinge on the luminescent material 50 to be converted into the further UV-radiation.

In the arrangement of the photochemical reactor 14 as shown in Fig. 2, the photochemical reactor 14 enables a clear distinction to be made between the ultraviolet radiation to which the fluid 30 is exposed and that to which the further fluid 35 is exposed. For example, the protective sheath 22 may not be translucent to the UV-radiation UV1 emitted by the light source 40 and only be at least partially translucent to the further UV-radiation UV2 emitted by the luminescent material 50. In such an arrangement, the fluid 30 will substantially only be exposed to the further UV-radiation UV2, while the further fluid 35 is exposed both to the UV-radiation UV1 emitted by the light source 40 and to part of the further UV-radiation UV2 emitted by the luminescent material 50.

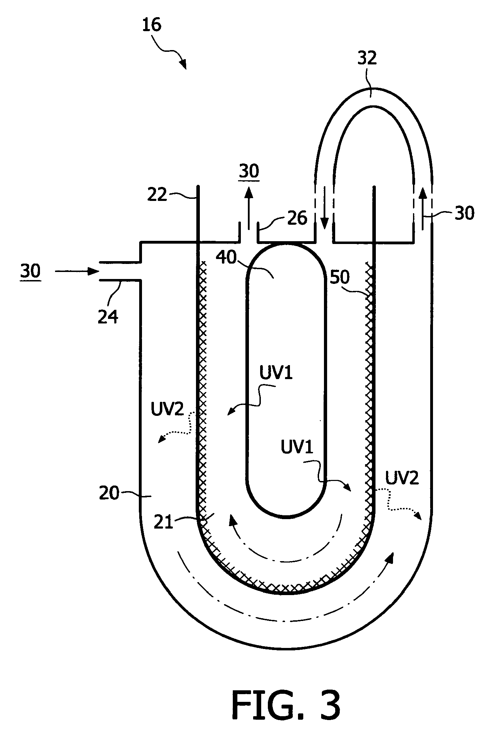

Fig. 3 shows a cross-sectional view of a still further embodiment of the photochemical reactor 16 according to the invention. In the photochemical reactor 16 shown in Fig. 3, the fluid 30 and the further fluid 30 are the same, both flowing successively through the vessel 20 and the additional vessel 21. The photochemical reactor 16 comprises the fluid inlet 24 via which the fluid 30 enters the photochemical reactor 16. Initially the fluid 30 flows through the vessel 20 and is exposed to the further UV-radiation UV2 emitted by the luminescent material 50. Typically this further UV-radiation UV2 has a reduced wavelength compared to the UV-radiation UV1 emitted by the light source 40 and thus the energy per photon is different. The further UV-radiation UV2 may be chosen to match the specific requirements of a first photochemical process to which the fluid 30 is initially exposed. The fluid 30 will subsequently flow through the connection means 32 to the additional vessel 21. The additional vessel 21 typically surrounds the light source 40 and mainly comprises UV-radiation UV1 emitted by the light source 40. The UV-radiation emitted by the light source 40 may be selected to match the specific requirements of a second photochemical process to which the fluid is subsequently exposed. As the luminescent material 50 generally emits the further UV-radiation UV2 in all directions, the further UV-radiation UV2 will also be present in the additional vessel 22, still enabling the first photochemical process to take place in the additional vessel. Thus, when use is made of the photochemical reactor 16 as shown in Fig. 3, a fluid 30 may successively be exposed to the further UV-radiation UV2 and the UV-radiation UV1, allowing two photochemical processes to be performed successively on the same fluid 30.

The connection means 32 between the vessel 20 and the additional vessel 21 may be formed by means of a connection hose 32 between the vessel 20 and the additional vessel 21. Alternatively, the connection means may be a hole (not shown) through the protective sheath 22 to connect the vessel 20 to the additional vessel 21. Also the shape of the photochemical reactor 16 may be different and may be optimized to enable an efficient flow of the fluid 30 through the photochemical reactor 16, for example, while reducing the pressure drop across the photochemical reactor 16. This may be beneficial, for example, when the photochemical reactor 16 is used for decontaminating water. Too large a pressure drop over the photochemical reactor 16 would reduce the water pressure too much, which is inconvenient for the water supply after the water has left the photochemical reactor 16.

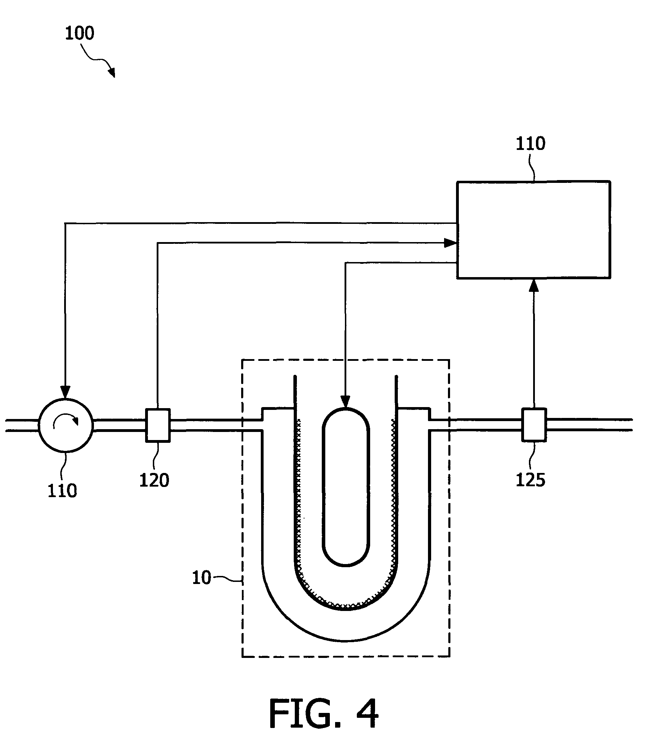

Fig. 4 shows a photochemical processing system 100 according to the invention. The photochemical processing system 100 comprises the photochemical reactor 10 as shown in Fig. 1A. The photochemical processing system 100 further comprises a processor 110. The processor 110 may be connected to the light source 40 for controlling an intensity of the UV-radiation UV1 emitted by the light source 40. The processor 110 may be connected to a pump 110 for controlling a speed at which the fluid 30 flows through the photochemical reactor 10. The processor 110 may also be connected to a first sensor 120 sensing, for example, a level of contamination of the fluid 30 before the fluid 30 enters the photochemical reactor 10 and to a second sensor 125 sensing, for example, a level of contamination of the fluid 30 after the fluid 30 has been exposed to the photochemical process. Using the intensity of the UV-radiation UV1, and/or the flowing speed of the fluid 30, the processor 110 may, for example, constantly monitor the quality of decontamination of the fluid 30 and may use these parameters to influence the decontamination. When, for example, the efficiency of the luminescent material 50 decreases, the decontamination of the fluid 30 is reduced. When this is measured by the processor 110, the processor 110 may reduce the flow speed of the fluid 30 to ensure a predefined decontamination level. Alternatively, when the contamination of the fluid 30 changes over time, the processor 110 may sense this and may adapt the illumination intensity of the light source 40 and/or may adapt the flow speed of the fluid to still provide the required decontamination.

Alternatively, other control means (not shown) may be present to influence the photochemical process of the photochemical processing system 100. For example, the flow speed of the fluid 30 may be controlled by a pressure valve (not shown) which may be used to reduce the flow of the fluid 30 when required. Also other sensors (not shown) may be present to sense the contamination of the fluid 30, or to sense the efficiency of the photochemical reactor 10, or to sense the intensity of the UV-radiation UV1 emitted by the light source 40, or to sense the intensity of the further UV-radiation UV2 emitted by the luminescent material 50.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements. In the device claim enumerating several means, several of these means may be embodied by one and the same item of hardware. The mere fact that certain measures are recited in mutually different dependent claims does not indicate that a combination of these measures cannot be used to advantage.

## Claims

1. A photochemical reactor (10, 12, 14, 16) for a fluid (30), the photochemical reactor (10, 12, 14, 16) comprising:
a vessel (20) comprising the fluid (30),
a light source (40) for generating UV-radiation (UV1) and emitting it towards the fluid (30), and
a luminescent material (50) arranged at least partially between the light source (40) and the fluid (30) for converting at least part of the UV-radiation (UV1) emitted by the light source (40) to further UV-radiation (UV2) having an increased wavelength compared to the UV-radiation (UV1),
the luminescent material (50) being removably connected to the photochemical reactor (10, 12, 14, 16) and being separate from the light source (40).

2. Photochemical reactor (10, 12, 14, 16) as claimed in claim 1, wherein the light source (40) is removably connected to the photochemical reactor (10, 12, 14, 16) and is separate from the luminescent material (50).

3. Photochemical reactor (10, 12, 14, 16) as claimed in claim 1 or 2, wherein the photochemical reactor (10, 12, 14, 16) comprises a luminescent screen (22, 52) comprising the luminescent material (50).

4. Photochemical reactor (10, 12, 14, 16) as claimed in claim 1 or 2, wherein a further fluid (30, 35) may flow between the light source (40) and the luminescent material (50).

5. Photochemical reactor (10, 12, 14, 16) as claimed in claim 3, wherein the further fluid (30) is identical to the fluid (30).

6. Photochemical reactor (10, 12, 14, 16) as claimed in any one of the preceding claims, wherein the fluid (30) flows through the photochemical reactor (10, 12, 14, 16).

7. Photochemical reactor (10, 12, 14, 16) as claimed in any one of the preceding claims, wherein the light source (40) is a dielectric barrier discharge lamp (40).

8. Photochemical reactor (10, 12, 14, 16) as claimed in any one of the preceding claims, wherein the light source (40) is arranged in a protective sheath (22) having a wall at least partially transparent to UV-radiation (UV1), the luminescent material (50) being arranged on the wall of the protective sheath (22).

9. Photochemical reactor (10, 12, 14, 16) as claimed in any one of the preceding claims, wherein the UV-radiation (UV1) comprises a wavelength below 200 nanometers and wherein the further UV-radiation (UV2) comprises a wavelength below 300 nanometers.

10. Photochemical processing system (100) for photochemically processing contamination in a fluid (30), the photochemical processing system (100) comprising the photochemical reactor (10, 12, 14, 16) as claimed in claims 1 to 9 and comprising a processor (110) for controlling an intensity of the light emitted by the light source (40) and/or for controlling a speed at which the fluid (30) flows through the photochemical reactor (10, 12, 14, 16) and/or for analyzing the contamination level of the fluid (30) before and/or after processing in the photochemical reactor (10, 12, 14, 16).

## Patentansprüche

1. Photochemischer Reaktor (10, 12, 14, 16) für eine Flüssigkeit (30), wobei der photochemische Reaktor (10, 12, 14, 16) Folgendes umfasst:
ein Gefäß (20) mit der Flüssigkeit (30),
eine Lichtquelle (40), um UV-Strahlung (UV1) zu erzeugen und zu der Flüssigkeit (30) hin zu emittieren, und
ein Leuchtmaterial (50), das zumindest teilweise zwischen der Lichtquelle (40) und der Flüssigkeit (30) angeordnet ist, um zumindest einen Teil der durch die Lichtquelle (40) emittierten UV-Strahlung (UV1) in weitere UV-Strahlung (UV2) mit einer erhöhten Wellenlänge im Vergleich zu der UV-Strahlung (UV1) umzuwandeln,
wobei das Leuchtmaterial (50) entfernbar mit dem photochemischen Reaktor (10, 12, 14, 16) verbunden ist und von der Lichtquelle (40) getrennt ist.

2. Photochemischer Reaktor (10, 12, 14, 16) nach Anspruch 1, wobei die Lichtquelle (40) entfernbar mit dem photochemischen Reaktor (10, 12, 14, 16) verbunden ist und von dem Leuchtmaterial (50) getrennt ist.

3. Photochemischer Reaktor (10, 12, 14, 16) nach Anspruch 1 oder 2, wobei der photochemische Reaktor (10, 12, 14, 16) einen Leuchtschirm (22, 52) umfasst, der das Leuchtmaterial (50) umfasst.

4. Photochemischer Reaktor (10, 12, 14, 16) nach Anspruch 1 oder 2, wobei eine weitere Flüssigkeit (30, 35) zwischen der Lichtquelle (40) und dem Leuchtmaterial (50) fließen kann.

5. Photochemischer Reaktor (10, 12, 14, 16) nach Anspruch 3, wobei die weitere Flüssigkeit (30) identisch mit der Flüssigkeit (30) ist.

6. Photochemischer Reaktor (10, 12, 14, 16) nach einem der vorhergehenden Ansprüche, wobei die Flüssigkeit (30) durch den photochemischen Reaktor (10, 12, 14, 16) fließt.

7. Photochemischer Reaktor (10, 12, 14, 16) nach einem der vorhergehenden Ansprüche, wobei die Lichtquelle (40) eine dielektrische Barriere-Entladungslampe (40) ist.

8. Photochemischer Reaktor (10, 12, 14, 16) nach einem der vorhergehenden Ansprüche, wobei die Lichtquelle (40) in einer Schutzhülle (22) mit einer für UV-Strahlung (UV1) zumindest teilweise durchlässigen Wand angeordnet ist, wobei das Leuchtmaterial (50) auf der Wand der Schutzhülle (22) angeordnet ist.

9. Photochemischer Reaktor (10, 12, 14, 16) nach einem der vorhergehenden Ansprüche, wobei die UV-Strahlung (UV1) eine Wellenlänge unter 200 Nanometern umfasst und wobei die weitere UV-Strahlung (UV2) eine Wellenlänge unter 300 Nanometern umfasst.

10. Photochemisches Bearbeitungssystem (100) zur photochemischen Bearbeitung von Kontamination in einer Flüssigkeit (30), wobei das photochemische Bearbeitungssystem (100) den photochemischen Reaktor (10, 12, 14, 16) nach den Ansprüchen 1 bis 9 umfasst und eine Bearbeitungseinheit (110) umfasst, um die Intensität des durch die Lichtquelle (40) emittierten Lichts zu steuern und/oder um eine Geschwindigkeit zu steuern, mit der die Flüssigkeit (30) durch den photochemischen Reaktor (10, 12, 14, 16) fließt und/oder um den Kontaminationsgrad der Flüssigkeit (30) vor und/oder nach der Bearbeitung im photochemischen Reaktor (10, 12, 14, 16) zu analysieren.

## Revendications

1. Réacteur photochimique (10, 12, 14, 16) pour un fluide (30), le réacteur photochimique (10, 12, 14, 16) comprenant :
une cuve (20) comprenant le fluide (30),
une source lumineuse (40) pour générer des rayonnements UV (UV1) et les envoyer vers le fluide (30), et
un matériau luminescent (50) disposé au moins partiellement entre la source lumineuse (40) et le fluide (30) pour convertir au moins une partie des rayonnements UV (UV1) émis par la source lumineuse (40) en autres rayonnements UV (UV12) ayant une longueur d'onde supérieure par rapport aux rayonnements UV (UV1),
le matériau luminescent (50) étant raccordé de manière amovible au réacteur photochimique (10, 12, 14, 16) et étant séparé de la source lumineuse (40).

2. Réacteur photochimique (10, 12, 14, 16) selon la revendication 1, dans lequel la source lumineuse (40) est raccordée de manière amovible au réacteur photochimique (10, 12, 14, 16) et est séparée du matériau luminescent (50).

3. Réacteur photochimique (10, 12, 14, 16) selon la revendication 1 ou 2, dans lequel le réacteur photochimique (10, 12, 14, 16) comprend un écran luminescent (22, 52) comprenant le matériau luminescent (50).

4. Réacteur photochimique (10, 12, 14, 16) selon la revendication 1 ou 2, dans lequel un autre fluide (30, 35) peut s'écouler entre la source lumineuse (40) et le matériau luminescent (50).

5. Réacteur photochimique (10, 12, 14, 16) selon la revendication 3, dans lequel le fluide ultérieur (30) est identique au fluide (30).

6. Réacteur photochimique (10, 12, 14, 16), selon l'une quelconque des revendications précédentes, dans lequel le fluide (30) s'écoule à travers le réacteur photochimique (10, 12, 14, 16).

7. Réacteur photochimique (10, 12, 14, 16) selon l'une quelconque des revendications précédentes, dans lequel la source lumineuse (40) est une lampe de décharge de bouclier diélectrique (40).

8. Réacteur photochimique (10, 12, 14, 16) selon l'une quelconque des revendications précédentes, dans lequel la source lumineuse (40) est disposée dans une gaine protectrice (22) ayant une paroi au moins partiellement transparente au rayonnement UV (UV1), le matériau luminescent (50) étant disposé sur la paroi de la gaine protectrice (22).

9. Réacteur photochimique (10, 12, 14, 16) selon l'une quelconque des revendications précédentes, dans lequel le rayonnement UV (UV1) comprend une longueur d'onde inférieure à 200 nanomètres et dans lequel le rayonnement UV ultérieur (UV2) comprend une longueur d'onde inférieure à 300 nanomètres.

10. Système de traitement photochimique (100) pour traiter photochimiquement la contamination dans un fluide (30), le système de traitement photochimique (100) comprenant le réacteur photochimique (10, 12, 14, 16) selon l'une quelconque des revendications 1 à 9, et comprenant un processeur (110) pour contrôler une intensité de la lumière émise par la source lumineuse (40) et/ou pour contrôler une vitesse à laquelle le fluide (30) s'écoule, à travers le réacteur photochimique (10, 12, 14, 16) et/ou pour analyser le niveau de contamination du fluide (30) avant et/ou après le traitement dans le réacteur photochimique (10, 12, 14, 16).
